# EUROPEAN PATENT SPECIFICATION

(11) **EP 2 895 309 B1**
(45) Date of publication and mention of the grant of the patent: **26.04.2017**
(21) Application number: 13747626.3
(22) Date of filing: 02.08.2013
(51) Int. Cl.: B29C 45/14, B29C 37/00

(54) **METHOD OF FORMING AN IMPLANTABLE MEDICAL DEVICE AND MEDICAL ELECTRICAL LEAD.**
VERFAHREN ZUM FORMEN EINER IMPLANTIERBAREN MEDIZINISCHEN VORRICHTUNG UND MEDIZINISCHER ELEKTRISCHER LEITER.
METHOD POU FABRIQUER UN APPAREIL MÉDICAL IMPLANTABLE AND CONDUCTEUR ÉLECTRIQUE MÉDICAL.

(30) Priority: 11.09.2012 US 201261699396 P
(43) Date of publication of application: 22.07.2015
(73) Proprietor: Cardiac Pacemakers, Inc., St. Paul, Minnesota 55112 (US)
(72) Inventor: ARNHOLT, Devon N., Shoreview, Minnesota 55126 (US); EGGERT, Joel T., Plymouth, Minnesota 55442 (US); BYRON, Mary M., Roseville, Minnesota 55113 (US); WULFMAN, David R., Minneapolis, Minnesota 55405 (US); PERREY, Christopher, Victoria, Minnesota 55386 (US)
(74) Representative: Pfenning, Meinig & Partner mbB
(86) International application number: PCT/US2013/053448
(87) International publication number: WO 2014/042779

(56) References cited:
- US-A1- 2010 241 204
- US-A1- 2011 054 581

## Description

### CROSS-REFERENCE TO RELATED APPLICATIONS

This application claims the benefit under 35 U.S.C. § 119(e) to U.S. Provisional Application No. 61/699,396, entitled "CONFORMAL POROUS THIN LAYER COATING AND METHOD OF MAKING", filed on September 11, 2012, which is herein incorporated by reference in its entirety.

### TECHNICAL FIELD

The present disclosure relates to medical devices, such as leads, having a body and a porous layer disposed on the body.

### BACKGROUND

Polymeric material such as silicone rubber, polyurethane, and other polymers are used as insulation materials for medical electrical leads. For cardiac rhythm management systems, such leads are typically extended intravascularly to an implantation location within or on a patient's heart, and thereafter coupled to a pulse generator or other implantable device for sensing cardiac electrical activity, delivering therapeutic stimuli, and the like. The leads are desirably highly flexible to accommodate natural patient movement, yet also constructed to have minimized profiles.

During and after implementation, the leads and lead body materials are exposed to various external conditions imposed, for example, by human muscular, skeletal and cardiovascular systems, body fluids, the pulse generator, other leads, and surgical instruments used during implantation and exploration procedures. Accordingly, there are ongoing efforts to identify lead body materials that are able to withstand a variety of conditions over a prolonged period of time while maintaining desirable flexibility characteristics and a minimized profile.

US 2011 / 0054581 A1 is directed to a medical electrical lead including an insulative lead body formed, at least in part, from polyisobutylene urethane, urea or urethane/urea copolymer. The lead body can also include an outer tubular insulator and/or an inner elongated member of said material. Furthermore, portions of the lead body can be either extruded or molded.

US 2010 / 0241204 A1 relates to an electrode and a method of making an electrode having a porous coating including a fiber mesh, a multi-layer coating and an outer coating. The various electrode coatings include pores in the coatings that prevent access by protein or cells while allowing for ion and/or liquid access.

### SUMMARY

Disclosed herein are various embodiments of an implantable medical device, as well as methods for forming the implantable medical device.
In Example 1, a method of forming an implantable medical device is provided. The method includes electrospinning or electrospraying a first material onto a substrate to form a fibrous matrix having pores. A second material is extruded or molded over the fibrous matrix so that the second material fills at least a portion of the pores of the fibrous matrix. After the second material is extruded or molded, the substrate is removed to form an implantable medical device with an inner surface, an outer surface and a lumen.
In Example 2, the implantable medical device according to Example 1, wherein after removal of the substrate, the fibrous matrix is on the inner surface of the implantable medical device.
In Example 3, the implantable medical device according to either Example 1 or Example 2, wherein the first material comprises a polyurethane or a fluoropolymer material.
In Example 4, the implantable medical device according to any of Examples 1-3, wherein the second material comprises silicone.
In Example 5, the implantable medical device according to any of Examples 1 and 3-4, wherein after removal of the substrate, the fibrous matrix is on the outer surface of the medical device.
In Example 6, the implantable medical device according to any of Examples 1-5, wherein the fibrous matrix and the second material in the pores form a layer and wherein the layer has a lower coefficient of friction than the second material.
In Example 7, the implantable medical device according to any of Examples 1-6, wherein the fibrous matrix and the second material in the pores form a layer and wherein the layer has a higher abrasion resistance than the second material.
In Example 8, the implantable medical device according to any of Examples 1-7, wherein the second material is cured after the extrusion or molding step.
In Example 9, a medical electrical lead includes an insulative body including a lumen extending through the insulative body, forming an inner surface eand an outer surface. A porous layer is disposed on the inner surface of the insulative body. and the porous layer includes a first material. The insulative body includes a second material, which is different from the first material.
In Example 10, the medical electrical lead of Example 9, wherein the second material comprises silicone.
In Example 11, the medical electrical lead of either Example 9 or Example 10, wherein the first material comprises at least one member selected from the group consisting of polyurethanes and fluoropolymer materials.
In Example 12, the medical electrical lead of any of Examples 9-11, wherein the porous layer is at least partially embedded in the insulative body.
In Example 13, the medical electrical lead of any of Examples 9-12, wherein the porous layer is an electrospun or electrosprayed layer.
In Example 14, the medical electrical lead of any of Examples 9-13, wherein the porous layer has a lower coefficient of friction than the insulative body.
In Example 15, the medical electrical lead of any of Examples 9-14, wherein the porous layer has a higher abrasion resistance than the insulative body.
In Example 16, the medical electrical lead of any of Examples 9-15, wherein the porous layer has a thickness of about 178 microns or less.
In Example 17, a method of forming an implantable medical device is provided. The method includes forming a porous layer of a first material on a substrate, extruding or molding a second material over the layer so that the second material fills at least a portion of the pores of the layer, and removing the substrate after extruding or molding the second material to form an implantable medical device with an inner surface, an outer surface and a lumen.
In Example 18, the method of Example 17, wherein the porous layer is on the inner surface of the implantable medical device.
In Example 19, the method of Example 17 or Example 18, wherein the layer is on the outer surface of the implantable medical device.
In Example 20, the method of any of Examples 17-19 wherein the step of forming a porous layer includes electrospinning or electrospraying the first material on a core pin or extrusion mandrel.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

FIG. 1 illustrates an exemplary implantable medical device.
FIG. 2 illustrates an alternative exemplary implantable medical device.
FIG. 3, FIG. 4 and FIG. 5 are alternative cross-sectional views of the exemplary implantable medical device of FIG. 2, taken along line 3 -3.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

A more complete understanding of the present invention is available by reference to the following detailed description of numerous aspects and embodiments of the invention. The detailed description of the invention which follows is intended to illustrate but not limit the invention.

In accordance with various aspects of the disclosure, a medical device includes a porous layer disposed on a second layer. The porous layer includes a first material and the second layer includes a second material. The first material and the second material can be the same or different. In certain aspects of the disclosure, the medical device can be a medical electrical device, such as a medical electrical lead.

Medical electrical devices typically include (a) an electronic signal generating component and (b) one or more leads. The electronic signal generating component can contain a source of electrical power (e.g., a sealed battery) and an electronic circuitry package, which produces electrical signals that are sent into the body (e.g., the heart, nervous system, etc.). Leads comprise at least one flexible elongated conductive member (e.g., a wire, cable, etc.), which is insulated along at least a portion of its length, generally by an elongated polymeric component often referred to as a lead body. The conductive member is adapted to place the electronic signal generating component of the device in electrical communication with one or more electrodes, which provide for electrical connection with the body. Leads are thus able to conduct electrical signals to the body from the electronic signal generating component. Leads may also relay signals from the body to the electronic signal generating component.

Examples of medical electrical devices include, for example, implantable electrical stimulation systems including neurostimulation systems such as spinal cord stimulation (SCS) systems, deep brain stimulation (DBS) systems, peripheral nerve stimulation (PNS) systems, gastric nerve stimulation systems, cochlear implant systems, and retinal implant systems, among others, and cardiac systems including implantable cardiac rhythm management (CRM) systems, implantable cardioverter-defibrillators (ICD's), and cardiac resynchronization and defibrillation (CRDT) devices, among others.

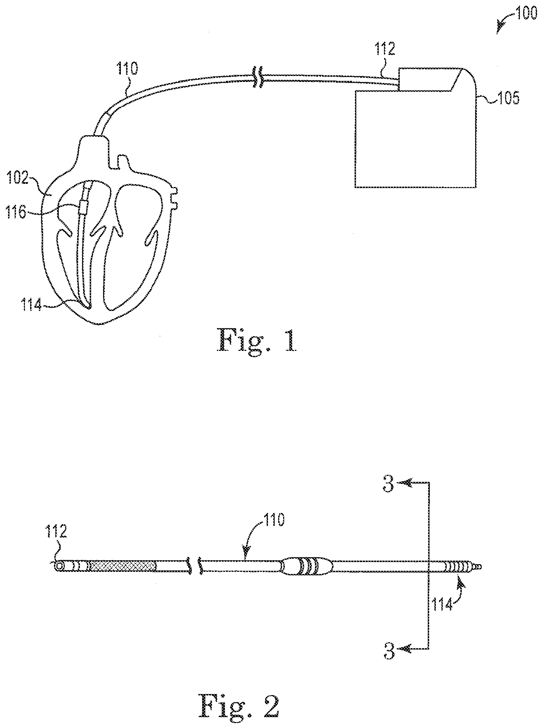

Fig. 1 is a schematic illustration of a lead system 100 for delivering and/or receiving electrical pulses or signals to stimulate, shock, and/or sense the heart 102. The lead system 100 includes a pulse generator 105 and a medical electrical lead 110. The pulse generator 105 includes a source of power as well as an electronic circuitry portion. The pulse generator 105 is a battery-powered device which generates a series of timed electrical discharges or pulses. The pulse generator 105 is generally implanted into a subcutaneous pocket made in the wall of the chest. Alternatively, the pulse generator 105 may be placed in a subcutaneous pocket made in the abdomen, or in another location. It should be noted that while the medical electrical lead 110 is illustrated for use with a heart 102, the medical electrical lead 110 is suitable for other forms of electrical stimulation/sensing as well.

The medical electrical lead 110 extends from a proximal end 112, where it is coupled with the pulse generator 105 to a distal end 114, which is coupled with a portion of a heart 102, when implanted or otherwise coupled therewith. An outer insulating lead body extends generally from the proximal end 112 to the distal end 114 of the medical electrical lead 110. Also disposed along a portion of the medical electrical lead 110, for example near the distal end 114 of the medical electrical lead 110, is at least one electrode 116 which electrically couples the medical electrical lead 110 with the heart 102. At least one electrical conductor (not shown) is disposed within the lead body and extends generally from the proximal end 112 to the distal end 114 of the medical electrical lead 110. The at least one electrical conductor electrically couples the electrode 116 with the proximal end 112 of the medical electrical lead 110. The electrical conductor carries electrical current and pulses between the pulse generator 105 and the electrode 116, and to and from the heart 102. In one option, the at least one electrical conductor is a coiled conductor. In another option, the at least one electrical conductor includes one or more cables. Typical lengths for such leads vary from about 35 cm to 40 cm to 50 cm to 60 cm to 70 cm to 80 cm to 90 cm to 100 cm to 110 cm to 120 cm, among other values. Typical lead diameters vary from about 4 to 5 to 6 to 7 to 8 to 9 French, among other values.

FIG. 2 is an alternative view of the medical electrical lead 110 which includes an elongated, insulative lead body extending from a proximal end 112 to a distal end 114.

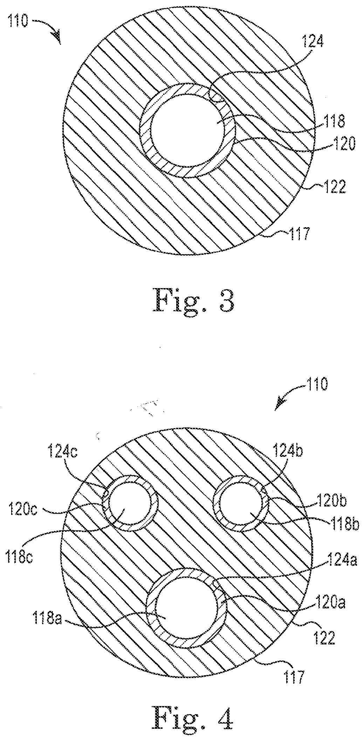

FIG. 3 shows an exemplary cross-sectional view of the medical electrical lead 110 of FIG. 2 as taken along line 3-3, and which includes an insulative body 117, a lumen 118 and a porous layer 120. In some embodiments, the lumen 118 extends through the medical electrical lead 110 and insulative body 117 from the proximal end 112 to the distal end 114. In some embodiments, the lumen 118 may have a small diameter. For example, the lumen 18 may have a diameter of about 127, 254, or 381 microns (0.005 inch, 0.010 inch or 0.015 inch) or a diameter of about 1016, 1143, or 1270 microns (0.040 inch, 0.045 inch or 0.050 inch) or may be within a range delimited by a pair of the foregoing values. In some embodiments, the lumen 118 may have a constant or substantially constant diameter along the length of the insulative body 117. In other embodiments, the diameter of the lumen 118 may vary along the length of the insulative body 117. For example, the diameter of the lumen 118 may decrease in a gradual or a stepped manner towards the distal end 114 or the proximal end 112. Although, the insulative body 117 and the lumen 118 are described as having a diameter and thus having a cylindrical shape, the insulative body 117 and the lumen 118 may have any suitable cross-sectional shape.

The insulative body 117 includes a flexible and/or stretchable material. In some embodiments, the insulative body 117 can include or primarily includes a polymeric material. Suitable materials for the insulative body 117 include silicone and homopolymers, copolymers and terpolymers of various polysiloxanes, polyurethanes, fluoropolymers, polyolefins, polyamides and polyesters. Suitable polyurethanes may include polycarbonate, polyether, polyester and polyisobutyelne (PIB) polyurethanes. Suitable PIB polyurethanes are disclosed in U.S. published application 2010/0023104, which is incorporated herein by reference in its entirety. Further examples of such copolymers and methods for their synthesis are generally described in WO 2008/060333, WO 2008/066914, U.S. Application No. 12/492,483 filed on June 26, 2009, entitled POLYISOBUTYLENE URETHANE, UREA AND URETHANE/UREA COPOLYMERS AND MEDICAL DEVICES CONTAINING THE SAME, and U.S. Application No. 12/874,887, filed September 2, 2010, and entitled Medical Devices Including Polyisobutylene Based Polymers and Derivatives Thereof, all of which are incorporated herein by reference in their entirety. Suitable fluoropolymer materials include polyvinylidene fluoride, polytetrafluoroethylene and expanded polytetrafluoroethylene.

In some embodiments, one or more electrodes may extend through the insulative body 117 and the lumen 118. The insulative body 117 can prevent the electrodes from contacting the surrounding tissue when the medical electrical lead 110 is implanted.

The insulative body 117 has an outer surface 122 and an inner surface 124. The outer surface 122 may be exposed to the surrounding tissue of a patient. Alternatively a coating may be applied to the outer surface 122 of the insulative body 117. For example, a coating may be applied to the outer surface 122 of the insulative body 117 to change the lubricity, abrasion resistance, dielectric strength, hydrophobicity, and/or other property of the insulative body 117.

In certain embodiments, the porous layer 120 may be on the inner surface 124 of the insulative body 117. In certain embodiments, the porous layer 120 may include a fibrous matrix of randomly aligned fibers formed by electrospinning or electrospraying and pores or spaces may be formed between the fibers. In certain embodiments, the fibers may include a polyurethane or a fluoropolymer material. Suitable polyurethanes include polyether, polyester and polyisobutylene (PIB) polyurethanes, as described herein with respect to the insulative body 117. Suitable fluoropolymer materials include polyvinylidene fluoride, ethylene tetrafluoroethlyene (ETFE), poly(vinylidene fluoride-co-hexafluoropropene) (PVDF) and expanded polytetrafluoroethylene.

As described further herein, the porous layer 120 may be at least partially embedded in the insulative body 117. For example, the material of the insulative body 117 may be present in at least a portion of the pores included in the porous layer 120. In some embodiments, the thickness of the insulative body 117 is sized such that porous layer 120 is not exposed at the outer surface of medical electrical lead 110. For example, a portion of the insulative body 117 may cover the outer surface of the porous layer 120.

In some embodiments, the material of the porous layer 120 may be selected to increase abrasion resistance, dielectric strength, hydrophobicity and/or the lubricity along the inner surface 124 of the insulative body 117. In some embodiments, the porous layer 120 may lower the coefficient of friction, creating a more lubricious surface on the insulative body 117. In other embodiments, the porous layer 120 may increase abrasion resistance of the insulative body 117. In still other embodiments, the porous layer 120 may include a higher dielectric material and may increase the dielectric strength of the insulative body 117. In still other embodiments, the porous layer 120 may increase or decrease the hydrophobicity of the insulative body 117.

The porous layer 120 may extend from the proximal end 112 to the distal end 114 of the medical electrical lead 110. In some embodiments, the porous layer 120 may extend the entire length of the insulative body 117. In other embodiments, the porous layer 120 may extend along a portion of the insulative body 117.

The medical electrical lead 110 may be formed by a layer transfer method which includes applying a first material onto a substrate followed by applying a second material onto the first material. The substrate may be removed after application of the second material to form the medical electrical lead 110.

The medical electrical lead 110 of Fig. 3 may be formed by applying a first material to the outer surface of a substrate, such as a core pin or extrusion mandrel, to form a porous layer. This porous layer may form the entire or a portion of the porous layer 120. In one example, the first material may be electro-spun or electrosprayed onto the outer surface of a core pin or an extrusion mandrel to form the fibrous matrix of the porous layer 120. The core pin or extrusion mandrel may be rotated while the first material is electro-spun onto the outer surface. Electrospinning and electrospraying of polyurethane and fluoropolymer materials are described in US provisional application 61/523,069 filed on August 12, 2012, entitled METHOD FOR COATING DEVICES USING ELECTROSPINNING AND MELT BLOWING and US provisional application 61/563,218 filed on November 23, 2011, entitled FIBROUS MATRIX COATING MATERIALS, all of which are incorporated herein by reference in their entirety.

In certain embodiments, the fibrous matrix may be formed by a plurality of randomly aligned electrospun or electrosprayed fibers. The fibers may have diameters in the range of about 10-3000 nanometers (nm), for example. The fiber diameter size may be measured by taking the average size of the fibers. In certain embodiments, the fibers may have an average diameter size less than about 800 nm, 750 nm, 725 nm, 700 nm, 600 nm, 500 nm or 400 nm. In other embodiments, the fiber matrix may be formed partially or completely with hollow fibers using modified electrospinning and meltblowing techniques. The fibrous matrix of the porous layer 120 may be porous and pores may be formed between the fibers.

The first material may form a conformal layer on the core pin or extrusion mandrel. For example, the core pin or extrusion mandrel may have a tapered or stepped geometry, and the first material may conform to the outer surface of the core pin or extrusion mandrel.

A second material can be molded or extruded over the first material. The second material can form the insulative body 117. At least a portion of the second material may also fill the spaces or pores in the porous layer 120. The second material can then be cured. In certain embodiments, the second material may fill at least a portion of the pores of the first material before the second material is cured. By filling at least a portion of the pores of the first material of the porous layer 120 with the second material before curing, the insulative body 117 can be mechanically locked to the porous layer 120.

The porous layer 120 may be a composite material of the first and second materials. For example, the porous layer 120 may include the first material as a porous layer and the second material may be present in at least a portion of the pores. In some embodiments, the first material and the second material may be different. That is, the first and second materials may have different compositions. In some examples, the first and second materials may include compounds from the same class (e.g., polyurethanes) but with different compositions and/or different physical properties, such as durometer. For example, the first material and the second material may both be polyurethanes and the first material may have a higher Shore strength than the second material. In other examples, the first and second materials may be compounds from different classes. For example, the first material may be a polyurethane and the second material may be a silicone. In this example, the porous layer 120 may be a composite of polyurethane and silicone and the insulative body 117 may include silicone. When the first and second materials are different, the porous layer 120 enables at least one material property of the inner surface 124 of the insulative body 117 to be different than that of the outer surface 122.

After the second material is molded or extruded, the core pin or extrusion mandrel may be removed. Removing the core pin or extrusion mandrel forms the lumen 118. For example, removing the core pin or extrusion mandrel may form medical electrical lead 110 including the insulative body 117 and the porous layer 120 adjacent the lumen 118. Because the second material can fill at least a portion of pores of the porous layer 120, the porous layer 120 can be embedded in the insulative body 117 and the porous layer 120 can transfer with the insulative body 117. In other words, the porous layer 120 does not remain on the core pin or extrusion mandrel after removal of the core pin or extrusion mandrel.

The porous layer 120 may form a conformal layer on the outer surface of the core pin or extrusion mandrel, and the extruded or molded second material can form a conformal layer on the porous layer 120. In this way, the porous layer 120 and insulative body 117 can have non-uniform geometries. For example, the lumen 118 through the insulative body 117 may have a tapered or stepped geometry and the porous layer 120 may conform to the non-uniform shape.

As described herein, in some embodiments, the porous layer 120 may be formed by electrospinning. In these embodiments, suitable materials for the porous layer 120 include a biocompatible polymeric material capable of being electrospun. In other embodiments, the porous layer 120 may be formed by electrospraying. In these embodiments, suitable materials for the porous layer 120 include any biocompatible polymeric material capable of being electrosprayed.

The porous layer 120 may have a thickness as little as 2.54, 12.7, or 25.4 microns (0.0001 inch, 0.0005 inch, or 0.001 inch) or as great as 76.2, 127, or 178 microns (0.003 inch, 0.005 inch or 0.007 inch) or may be within a range delimited by a pair of the foregoing values. In some embodiments, the porous layer 120 has a thickness chosen such that the porous layer 120 has little to no effect on the stiffness or flexibility of the medical electrical lead 110 as compared to a medical electrical lead 110 without a porous layer 120. In some embodiments, porous layer 120 may be thinner than insulative body 117. For example, in some embodiments, the wall thickness of the insulative body 117 may be as thin as 25.4, 50.8, 76.2 or 101.6 microns (0.001 inch, 0.002 inch, 0.003 inch or 0.004 inch) or as thick as 178, 203, 229 or 254 microns (0.007 inch, 0.008 inch, 0.009 inch or 0.010 inch) or may be within a range delimited by a pair of the foregoing values.

In certain embodiments, the porous layer 120 may have a coefficient of friction that is less than that of the insulative body 117. In such embodiments, the porous layer 120 can increase the lubricity of respective surfaces of the insulative body 117. In certain embodiments, the coefficient of friction can be determined by the procedure described in ASTM G115.

In certain embodiments, the porous layer 120 may have a greater resistant to abrasion than that of the insulative body 117. In these embodiments, the porous layer 120 increases the abrasion resistance of the respective surface of the insulative body 117. An increased abrasion resistance may prevent the conductor from breaking through the insulative body 117 and contacting tissue surrounding the insulative body 117. In certain embodiments, the abrasion resistance of a material may be measured by the procedure described in ASTM D1894.

In certain embodiments, the porous layer 120 may have a different dielectric strength than that of the insulative body 117. For example, the porous layer 120 may have a dielectric constant that is greater than that of the insulative body 117.

In certain embodiments, the porous layer 120 may have a different hydrophobicity than that of the insulative body 117. In certain embodiments, the porous layer 120 may have a lower hydrophobicity than that of the insulative body 117. In other embodiments, the porous layer 120 may have a higher hydrophobicity than that of the insulative body 117.

In some embodiments, the second material may only be present in the pores or spaces of the porous layer 120. In these embodiments, the porous layer 120 and insulative body 117 do not exist as discrete, independent layers.

Figure 4 shows an alternative cross-sectional view of the medical electrical lead 110 which includes multiple lumens, first lumen 118a, second lumen 118b, and third lumen 118c, which may extend through the insulative body 117 from the proximal end 112 to the distal end 114. Similar to the embodiment shown in Figure 3, porous layers 120a, 120b, and 120c may be formed on inner surfaces 124a, 124b, and 124c, respectively, of the insulative body 117. The porous layers 120a, 120b, and 120c may have compositions different than that of the insulative body 117. Additionally, the porous layers 120a, 120b, and 120c may have the same composition or different compositions than one another. The porous layers 120a, 120b and 120c may be substantially similar to the porous layer 120 described herein.

The medical electrical lead 110 of Figure 4 may be formed as described above except porous layers 120a, 120b, and 120c may be formed on separate core pins or extrusion mandrels, the core pins or extrusion mandrels are arranged and the second material is extruded or cast over the arranged core pins or extrusion mandrels to form the insulative body 117.

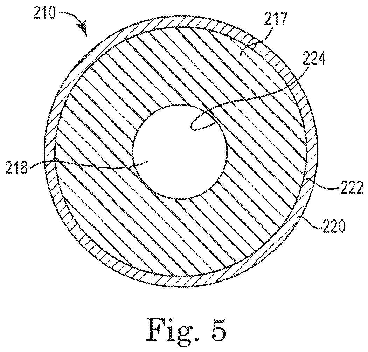

Figure 5 is a cross-sectional view of a still further alternative medical electrical lead 210 which includes a porous layer 220 on an outer surface 222 of an insulative body 217. The insulative body 217 and porous layer 220 are similar to insulative body 117 and porous layer 120, respectively.

The medical electrical lead 210 may be formed by applying a first material onto the interior surface of a substrate, such as a mold cavity, to form the porous layer of the porous layer 220. In certain examples, the first material may be electrospun or electrosprayed onto the interior surface of the mold cavity to form a fibrous matrix. The fibrous matrix formed may be by a plurality of randomly aligned fibers as described herein, and pores may be formed between the fibers.

A second material is then introduced into the mold cavity. The second material may fill at least a portion of the pores of the porous layer 220. The second material is than cured. The porous layer 220 may be a composite of the first material and the second material. For example, the porous layer 220 may include a porous layer of the first material and the second material may be present in at least a portion of the pores of the porous layer 220. In certain embodiments, the porous layer 220 may be embedded on the outer surface 222 of the insulative body 217 due to the presence of the second material in at least a portion of the pores prior to curing.

In certain embodiments, the substrate may include irregular surfaces. For example, surfaces of the mold cavity may not be smooth or may include a step, taper, channel or another surface feature resulting in the medical electrical lead 210 having a non-uniform thickness in the axial direction. The first material conforms to the surface of the mold regardless of the shape of the surface and the method described herein enables the formation of a conformal, porous layer 220 on the outer surface 222 of the insulative body 217, including when the outer surface 222 has an irregular topography.

Various modifications and additions can be made to the exemplary embodiments discussed without departing from the scope of the present invention. For example, while the embodiments described above refer to particular features, the scope of this invention also includes embodiments having different combinations of features and embodiments that do not include all of the described features. Accordingly, the scope of the present invention is intended to embrace all such alternatives, modifications, and variations as fall within the scope of the claims, together with all equivalents thereof.

The subject-matter of the present invention relates, inter alia, to the following aspects:
1. A method of forming an implantable medical device, the method comprising:
   electrospinning or electrospraying a first material onto a substrate to form a fibrous matrix having pores;
   extruding or molding a second material over the fibrous matrix so that the second material fills at least a portion of the pores of the fibrous matrix; and
   removing the substrate after extruding or molding the second material to form an implantable medical device with an inner surface, an outer surface and a lumen.
2. The method of aspect 1, wherein after removal of the substrate, the fibrous matrix is on the inner surface of the implantable medical device.
3. The method of aspect 1, wherein after removal of the substrate, the fibrous matrix is on the outer surface of the implantable medical device.
4. The method of any of aspects 1-3, wherein the fibrous matrix and the second material in the pores form a layer and wherein the layer has a lower coefficient of friction than the second material.
5. The method of any of aspects 1-4, wherein the fibrous matrix and the second material in the pores form a layer and wherein the layer has a higher abrasion resistance than the second material.
7. The method of any of aspects 1-5, wherein the second material is cured after the extrusion or molding step.
8. A medical electrical lead comprising:
   an insulative body, including a lumen extending through the insulative body, forming an inner surface and an outer surface; and
   a porous layer disposed on the inner surface of the insulative body, wherein the porous layer comprises a first material and the insulative body comprises a second material different from the first material
9. The medical electrical lead of aspect 8, wherein the porous layer is at least partially embedded in the insulative body.
10. The medical electrical lead of any of aspects 8-9, wherein the porous layer is an electrospun or electrosprayed layer.
11. The medical electrical lead of any of aspects 8-10, wherein the porous layer has a lower coefficient of friction than the insulative body.
12. The medical electrical lead of any of aspects 8-11, wherein the porous layer has a higher abrasion resistance than the insulative body.
13. The medical electrical lead of any of aspects 8-12, wherein the porous layer has a thickness of about 178 microns or less.
14. The medical electrical lead of any of aspects 1-13, wherein the second material comprises silicone.
15. The medical electrical lead of any of aspects 1-14, wherein the first material comprises at least one member selected from the group consisting of polyurethanes and fluoropolymer materials.

## Claims

1. A method of forming an implantable medical device, the method comprising:
electrospinning or electrospraying a first material onto a substrate to form a fibrous matrix having pores;
extruding or molding a second material over the fibrous matrix so that the second material fills at least a portion of the pores of the fibrous matrix; and
removing the substrate after extruding or molding the second material to form an implantable medical device with an inner surface, an outer surface and a lumen.

2. The method of claim 1, wherein after removal of the substrate, the fibrous matrix is on the inner surface of the implantable medical device.

3. The method of claim 1, wherein after removal of the substrate, the fibrous matrix is on the outer surface of the implantable medical device.

4. The method of any of claims 1-3, wherein the fibrous matrix and the second material in the pores form a layer and wherein the layer has a lower coefficient of friction than the second material.

5. The method of any of claims 1-4, wherein the fibrous matrix and the second material in the pores form a layer and wherein the layer has a higher abrasion resistance than the second material.

6. The method of any of claims 1-5, wherein the second material is cured after the extrusion or molding step.

7. An implantable medical electrical lead (110) comprising:
an insulative body (117), including a lumen (118) extending through the insulative body (117), forming an inner surface (124) and an outer surface (122); and
a porous layer (120) disposed on the inner surface (124) of the insulative body (117), wherein the porous layer (120) comprises a first material and the insulative body (117) comprises a second material different from the first material, and wherein the porous layer (120) is an electrospun or electrosprayed layer.

8. The medical electrical lead (110) of claim 7, wherein the porous layer (120) is at least partially embedded in the insulative body (117).

9. The medical electrical lead (110) of any of claims 7-8, wherein the porous layer (120) has a lower coefficient of friction than the insulative body (117).

10. The medical electrical lead (110) of any of claims 7-9, wherein the porous layer (120) has a higher abrasion resistance than the insulative body (117).

11. The medical electrical lead (110) of any of claims 7-10, wherein the porous layer (120) has a thickness of about 178 microns or less.

12. The medical electrical lead (110) of any of claims 7 -11, wherein the second material comprises silicone.

13. The medical electrical lead (110) of any of claims 7 -12, wherein the first material comprises at least one member selected from the group consisting of polyurethanes and fluoropolymer materials.

## Patentansprüche

1. Verfahren zum Bilden einer implantierbaren medizinischen Vorrichtung, welches Verfahren aufweist:
Elektrospinnen oder Elektrosprühen eines ersten Materials auf ein Substrat, um eine Fasermatrix mit Poren zu bilden;
Extrudieren oder Formen eines zweiten Materials über der Fasermatrix, so dass das zweite Material zumindest einen Teil der Poren der Fasermatrix füllt; und
Entfernen des Substrats nach dem Extrudieren oder Formen des zweiten Materials, um eine implantierbare medizinische Vorrichtung mit einer inneren Oberfläche, einer äußeren Oberfläche und einem Lumen zu bilden.

2. Verfahren nach Anspruch 1, bei dem nach dem Entfernen des Substrats die Fasermatrix auf der inneren Oberfläche der implantierbaren medizinischen Vorrichtung ist.

3. Verfahren nach Anspruch 1, bei dem nach dem Entfernen des Substrats die Fasermatrix auf der äußeren Oberfläche der implantierbaren medizinischen Vorrichtung ist.

4. Verfahren nach einem der Ansprüche 1 - 3, bei dem die Fasermatrix und das zweite Material in den Poren eine Schicht bilden, und bei dem die Schicht einen geringeren Reibungskoeffizienten als das zweite Material hat.

5. Verfahren nach einem der Ansprüche 1 - 4, bei dem die Fasermatrix und das zweite Material in den Poren eine Schicht bilden, und bei dem die Schicht einen höheren Abriebwiderstand als das zweite Material hat.

6. Verfahren nach einem der Ansprüche 1-5, bei dem das zweite Material nach der Extrusion oder dem Formungsschritt gehärtet wird.

7. Implantierbare medizinische elektrische Leitung (110), welche aufweist:
einen isolierenden Körper (117) enthaltend ein Lumen (118), das sich durch den isolierenden Körper (117) erstreckt, der eine innere Oberfläche (124) und eine äußere Oberfläche (122) bildet; und
eine poröse Schicht (120), die auf der inneren Oberfläche (124) des isolierenden Körpers (117) angeordnet ist, wobei die poröse Schicht (120) ein erstes Material aufweist und der isolierende Körper (117) ein zweites Material, das von dem ersten Material verschieden ist, aufweist, und wobei die poröse Schicht (120) eine elektrogesponnene oder elektrogesprühte Schicht ist.

8. Medizinische elektrische Leitung (110) nach Anspruch 7, bei der die poröse Schicht (120) zumindest teilweise in den isolierenden Körper (117) eingebettet ist.

9. Medizinische elektrische Leitung (110) nach einem der Ansprüche 7 - 8, bei der die poröse Schicht (120) einen niedrigeren Reibungskoeffizienten als der isolierende Körper (117) hat.

10. Medizinische elektrische Leitung (110) nach einem der Ansprüche 7 - 9, bei der die poröse Schicht (120) einen höheren Abriebwiderstand als der isolierende Körper (117) hat.

11. Medizinische elektrische Leitung (110) nach einem der Ansprüche 7 - 10, bei der die poröse Schicht (120) eine Dicke von etwa 178 Mikrometer oder weniger hat.

12. Medizinische elektrische Leitung (110) nach einem der Ansprüche 7 - 11, bei der das zweite Material Silikon aufweist.

13. Medizinische elektrische Leitung (110) nach einem der Ansprüche 7 - 12, bei der das erste Material zumindest ein Teil aufweist, das aus der Gruppe bestehend aus Polyurethan- und Fluorpolymermaterialien ausgewählt ist.

## Revendications

1. Procédé de formation d'un dispositif médical implantable, le procédé comprenant :
l'électro-filage ou l'électro-pulvérisation d'un premier matériau sur un substrat afin de former une matrice fibreuse comportant des pores;
l'extrusion ou le moulage d'un second matériau sur la matrice fibreuse de telle sorte que le second matériau remplisse au moins une partie des pores de la matrice fibreuse ; et
l'enlèvement du substrat après extrusion ou moulage du second matériau afin de former un dispositif médical implantable comportant une surface interne, une surface externe et une lumière.

2. Procédé selon la revendication 1, dans lequel, après l'enlèvement du substrat, la matrice fibreuse est sur la surface interne du dispositif médical implantable.

3. Procédé selon la revendication 1, dans lequel, après l'enlèvement du substrat, la matrice fibreuse est sur la surface externe du dispositif médical implantable.

4. Procédé selon l'une quelconque des revendications 1-3, dans lequel la matrice fibreuse et le second matériau dans les pores forment une couche et dans lequel la couche présente un coefficient de friction inférieur à celui du second matériau.

5. Procédé selon l'une quelconque des revendications 1-4, dans lequel la matrice fibreuse et le second matériau dans les pores forment une couche et dans lequel la couche présente une résistance à l'abrasion supérieure à celle du second matériau.

6. Procédé selon l'une quelconque des revendications 1-5, dans lequel le second matériau est durci après l'étape d'extrusion ou de moulage.

7. Connexion électrique médicale implantable (110) comprenant :
un corps isolant (117), incluant une lumière (118) qui s'étend au travers du corps isolant (117), en formant une surface interne (124) et une surface externe (122) ; et
une couche poreuse (120) disposée sur la surface interne (124) du corps isolant (117), dans laquelle la couche poreuse (120) comprend un premier matériau et le corps isolant (117) comprend un second matériau différent du premier matériau, et dans laquelle la couche poreuse (120) est une couche électro-filée ou électro-pulvérisée.

8. Connexion électrique médicale (110) selon la revendication 7, dans laquelle la couche poreuse (120) est au moins partiellement noyée dans le corps isolant (117).

9. Connexion électrique médicale (110) selon l'une quelconque des revendications 7-8, dans laquelle la couche poreuse (120) présente un coefficient de friction inférieur à celui du corps isolant (117).

10. Connexion électrique médicale (110) selon l'une quelconque des revendications 7-9, dans laquelle la couche poreuse (120) présente une résistance à l'abrasion supérieure à celle du corps isolant (117).

11. Connexion électrique médicale (110) selon l'une quelconque des revendications 7-10, dans laquelle la couche poreuse (120) présente une épaisseur d'environ 178 micromètres ou moins.

12. Connexion électrique médicale (110) selon l'une quelconque des revendications 7-11, dans laquelle le second matériau comprend du silicone.

13. Connexion électrique médicale (110) selon l'une quelconque des revendications 7-12, dans laquelle le premier matériau comprend au moins un élément choisi parmi le groupe constitué par les polyuréthanes et les matériaux à base de polymères fluorés.
